# EUROPEAN PATENT APPLICATION

(11) **EP 1 275 725 A1**
(43) Date of publication of application: **15.01.2003**
(21) Application number: 01911773.8
(22) Date of filing: 16.03.2001
(51) Int. Cl.: C12N 15/74, C12N 15/53, C07K 14/335

(54) **USE OF THE NUCLEOTIDE SEQUENCE OF THE LACTOBACILLUS CASEI LACTOSE OPERON PROMOTER FOR REGULATING GENE EXPRESSION BY MEANS OF AN ANTITERMINATOR PROTEIN**

(30) Priority: 07.04.2000 ES 200000897
(71) Applicant: CONSEJO SUPERIOR DE INVESTIGACIONES CIENTIFICAS, 28006 Madrid (ES)
(72) Inventor: Gosalbes Soler M. J. Insto.Agro. y Tecn. de Alimen, 46100 Burjassot (Valencia) (ES); Peres Arellano, Isabel Insto.Agro. y Tecn.de Alim., 46100 Burjassot (Valencia) (ES); Esteban Nieto, Carlos D. I.A. y T. de Alimento, 46100 Burjassot (Valencia) (ES); Galan Asuncion, José L. I. A. y T. de Alimento, 46100 Burjassot (Valencia) (ES); Perez Martinez, Gaspar I. A. y T. de Alimentos, 46100 Burjassot (Valencia) (ES)
(74) Representative: Ungria Lopez, Javier
(86) International application number: ES0100104
(87) International publication number: WO01077346

(57) **Abstract**

This invention describes the use of a method for the expression of proteins in bacteria, which is based on the inducer effect, for example, of a carbohydrate on the anti-terminating activity of a protein of the family of anti-terminators, BlgG/SacY. The procedure has been tested with the system derived from the operon *lacTEGF*, inducible by lactose in *Lactobacillus casei,* for the expression of proteins of different kinds. In the invention are included examples of expression in multicopy plasmids or of integration in the chromosomal operon *lacTEGF*, thus achieving the coordinated expression of the desired genes together with those that code the enzymes of the metabolism of the lactose.

## Description

### FIELD OF THE TECHNIQUE

The procedure of genic expression described herein is included within the Biotechnology sector. This invention is geared towards both the food industry, for example the dairy industry, as well as the pharmaceutical industries, as it makes it possible to express proteins in *Lactobacillus casei* of high nutritional, clinical and immunological value.

### STATE OF THE ART

In the last decade, a number of systems of control of the expression of fragments of DNA for bacteria have been developed, and specifically, for bacteria of lactic acid (BAL) of industrial interest [W.M. de Vos, 1999, International Dairy Journal 9:3-10]. However, in the majority of these cases, *Lactococcus lactis* was taken as the BAL model, and the results were not always transferable to other lactic bacteria such as the *Lactobacillus.* The different systems of control of the genic expression developed are based on expression and integration vectors, in which different marker genes and regulatory elements are used. However, on many occasions, these vectors lack regulatory elements of their own or they come from other microorganisms, thus rendering the regulation of the genes expressed difficult. Recently, an expression vector based on the regulatory elements of the gene *nisA* of *Lactococcus lactis,* which codes an antimicrobial peptide, nisin, has been developed [Ruyter P.G.G.A., Kuipers O. and de Vos W.M., 1996, Appl. Environm. Microbiol. 62:3662-3667, EP0712935, 1996-05-22, Stichting NL I Zuivelonderzoek]. This system makes it possible to increase the expression of any gene more than 1000 times in a controlled manner. However, the process of induction occurs by a mechanism for the transducing of a signal of the two-component regulation system type, thus requiring the presence of the products of the genes *nisK* and *nisR*, for which reason this system requires several manipulation steps in order to transfer to other microorganisms lacking the regulator genes. Another system of expression in *Lactococcus lactis* is the one based on the RNA polymerase of the T7 phage of *E. coli* (GB2278358, 1994-11-30, Lynxvale LTD (GB)). This technique has been used to obtain the expression of proteins of an immunological nature in *L. lactis*. However, this system of expression involves the use of heterological DNA as the promoter and structural gene of the RNA polymerase of the T7 phage.

Also, the use of marker genes that permit the metabolism of sugars little used by lactic acid bacteria [Leenhouts K, Bolhuis A., Venema G. and Kok J., 1998, Appl. Microbiol and Biotechnol 49, 417-423] or of those that express peptides with an antimicrobial activity, requires the addition of the respective inducers to the culture medium in order to maintain the expression, thus involving additional cost.

Any bacterium is capable of using different carbohydrates as the sole source of carbon and energy. The genes'that code the enzymes involved in the transport and metabolism of sugars are expressed, in the majority of cases, solely if the relevant carbohydrate is present in the medium and if the latter lacks easily metabolizable sugars, such as glucose and fructose. This kind of regulation makes reference to mechanisms of induction and catabolic repression, respectively.

The easily assimilated sugars are always consumed by the bacterial cells on a preferential basis, in such as way that the expression of the genes of the catabolism of other sources of carbon becomes attenuated or inhibited, which is called catabolic repression. This process has been studied extensively both in Gram-negative as well as Gram-positive bacteria, with the system of phosphotransferase dependent on the phosphoenolpyruvate (PTS) being involved in all of them. In Gram-positive bacteria, it has been established that in the presence of glucose, the phosphorylated protein HPr in the serine 46 is bonded to a repressor protein called CcpA, and the complex formed recognizes a highly preserved DNA sequence (cre sequence) which tends to be located in the promoter zone and blocks the start of the transcription of the genes affected.

Also, the operons of the catabolism of sugars are often regulated by means of activator, repressor or anti-terminator proteins, so that the transcription and translation of their genes is effected in the presence of the inducer. There are examples of induction by anti-termination in Gram-negative bacteria such as *Escherichia coil, Erwinia chrysanthemi, Klebsiella pneumoniae* and *Klebsiella oxytoca* which have already been studied. Among the Gram-positive bacteria, *Bacillus subtilis* is the most thoroughly studied, with 7 different anti-termination systems having been described. This type of regulation mechanism has also been found in *B. amyloliquefaciens, B. stearothermophilus, Staphylococcus carnosus, Lactococcus lactis* and *Lactobacillus casei.* The anti-termination mechanisms are characterized by presenting an RAT sequence of bonding to the RNA, which is recognized by the anti-terminator protein, and a terminator structure, which is located between the start of the transcription and that of the translation of the operon. In the presence of the inducer, the anti-terminator protein is bonded to the RAT sequence, destabilizes the terminator structure and allows the complete transcription of the operon. All of the anti-terminator proteins share a high degree of similarity, with three domains being identified in them: one binding to the RNA (RBD) and two regulated by the elements of the PTS (PRD-I and PRD-II). A high degree of homology has been observed in the RAT sequences of the different systems and it has even been found that the anti-terminator proteins of *B. subtilis* and *E. coli* are interchangeable, indicating that this system of regulation is kept in the different types of bacteria [Rutberg B., 1997, Molecular Microbiol. 23:413-421; Stülke J., Arnaud M., Rapoport G. and Martin-Verstraete, I., 1998, Molecular Microbiol. 28:865-874]. The role played by the elements of the PTS, EII and HPr, varies from one system to another but is based generally on the regulation of the anti-terminator activity by phosphorylation of the domains PRD-I and PRD-II. These PRD domains, however, are not required for the anti-terminator activity, as solely the 55-60 amino acids of the amino-terminal end are sufficient in order to break down the structure of the terminator and, thus, to induce the genic expression [Tortosa P., Aymerich S., Lindner C., Saier M.H.Jr., Reizer J. and D. LeCoq, 1997, J. Biol Chem. 272: 17230-17237].

One of the best-studied systems in BAL is the transport of lactose. In *Streptococcus thermophilus,* this sugar has been described as entering through a permease of an anti-transporter type, LacS, being hydrolyzed by a β-galactosidase, lacZ, a glucose and galactose, which is expelled to the external medium. The genes that code these protein are forming an operon, *lacSZ*, the expression of which is induced by galactose and repressed by catabolite [Poolman B., Royer T.J., Mainzer S.E. and Schmidt B.F., 1989, J. Bacteriol. 171:244-253; Poolman B., Royer T.J., Mainzer S.E. and Schmidt B.F., 1990, J. Bacteriol 172:4037-4047, Poolman B., 1993, FEMS Microniol. Rev. 12, 125-147]. Based on this system, mutants were obtained by integration between the *lacS* and *lacZ* genes, expressing a gene of resistance to chloramphenicol (*cat*) with a pattern of expression similar to that of the operon. However, the β-galactosidase (LacZ) activity was affected in the mutant strains possibly due to the fact that the binding site to the ribosome of the gene *lacZ* was lost [Mollet B., Knol J., Poolman B., Marciset O., Delley M., 1993, J. Bacteriol. 175:4315-4324, US5491079, 1999-02-13, Nestec S.A.].

The system of use of the lactose in *L. lactis* has been studied in detail [de Vos W.M. and Vaughan E.E., 1994, FEMS Microbiol. Rev. 15:217-239] and is made up by the operon *lacABCDFEG*, which codes the enzymes necessary for the utilization of the tagatose and the lactose. As in the case of the lactose genes of *Streptococcus mutans* or *Staphylococcus aureus,* the promoter located ahead of the gene *lacA* is regulated by the protein LacR, homologous to repressors such as GutR, FucR or DeoR of *E. coli*, with the inducer of the system being the tagatose 6-phosphate [van Rooijen R.J., and de Vos W.M., 1990, J. Biol. Chem. 265:18499-18503]. This inducible system has been used in the development of different expression vectors for *Lactococcus* [MacCormick, C.A., Griffin H.G. and Gasson M.J., 1995, FEMS Microbiol. Lett. 127:105-109; Platteeuw C., van Alen-Boerrigter I., van Schalkwijk S. and de Vos W.M., 1996, Appl. Environ. Microbiol. 62:1008-1013; EP0355036, 1990-02-21, NL Zuivelonderzoek Inst.].

However, the system of lactose use by *Lactobacillus casei* differs from that used by the two BALs mentioned earlier, both in the genic structure (*lacTEGF*) as well as in the regulation mechanism [Chassy, B. and Thompson J., 1983, J. Bacteriol. 154:1195-1203; Alpert, C.-A. and Chassy B.M., 1988, Gene 62:277-288; Porter E.V. and Chassy B.M., 1988, Gene 62:263-276; Alpert, C.-A and Chassy B.M., 1990, J. Biol. Chem. 265:22561-22568; Veyrat A., Monedero V. and Pérez-Martínez G., 1994, Microbiology 140, 1141-1149: Gosalbes M.J., Monedero V., Alpert, C.-A. and Pérez-Martínez G., 1997, FEMS Microbiol. Lett. 148:83-89] as it is induced thanks to an anti-terminator protein, LacT, homologous to proteins that regulate the expression of promoters of genes for the use of mono and disaccharides in other bacteria, such as BglG in *E. coil* or SacY in *Bacillus subtilis* [Alpert, C.-A. and Siebers U., 1997, J. Bacteriol. 179:1555-1562]. In *Lactobacillus casei* the inducer is the lactose, the only sugar present in milk and the majority sugar in mediums derived from it, through a mechanism of anti-termination in which different regulatory elements are involved, such as the sequence called RAT, which is the binding site to the RNA of anti-terminator proteins, a structure terminating the transcription and the anti-terminator protein [Gosalbes M.J. , Monedero V. and Pérez-Martínez G., 1999, J. Bacteriol 181:3928-3934]. Moreover, there is a strong repression by glucose mediated by the protein CcpA and elements of the phosphotransferase system dependent on the phosphoenolpyruvate (PTS) [Monedero V., Gosalbes M.J. and Pérez-Martínez G., 1997, J. Bacteriol. 179:6657-6664]. This regulation mechanism permits a strict and consistent control of the expression of the desired characteristics.

This system of regulation by anti-termination has been described both in Gram-negative as well as Gram-positive bacteria, which, as commented upon earlier, can be interchangeable between different operons in certain cases, and even in other bacteria [Manival X., Yang Y., Strub, M.P., Kochoyan, M., Steinmetz, M., and Aymerich, S. 1999, EMBO J. 16: 5019-5029]. However, it is important to note that this type of regulatory elements has not been used previously in the development of systems of expression in bacteria.

### DESCRIPTION OF THE INVENTION.

### Brief Description

This invention describes the use of a method for the expression of proteins in bacteria, which is based on the inducing effect, for example, of a carbohydrate on the anti-terminating activity of a protein from the family of anti-terminators BlgG/SacY. The procedure has been tested with the system derived from the operon *lacTEGF*, inducible by lactose in *Lactobacillus casei,* for the expression of proteins of different kinds. In the invention are included examples of expression in multicopy or integration plasmids in the chromosomal operon *lacTEGF,* thus achieving the coordinated expression of the desired genes together with those that code the enzymes of the metabolism of the lactose.

### Detailed Description

The system referred to by the inventors is based on the anti-terminating activity of LacT, which mediates the induction by lactose in *Lactobacillus casei.*

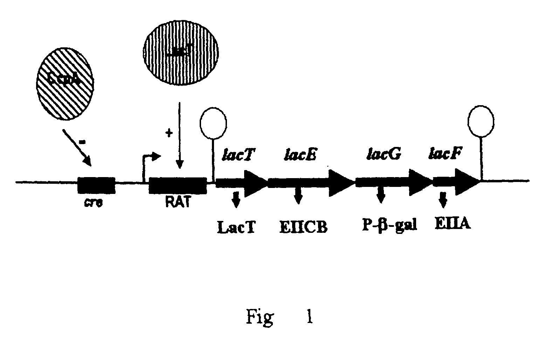

Figure 1 describes the genic structure and the regulatory elements of the lactose operon of *Lactobacillus casei* CECT5275. In this microorganism the operon of the lactose is located in the chromosome and consists of four *lacTEGF* genes that code an anti-terminator protein LacT, the element EIIBC of the transport of lactose by PTS, the phospho-β-galactosidase and the element EIIA of the PTS transport, respectively. The promoter with the regions -35 (TTTACA) and -10 (TACAAC) is located on the end 5' of this operon, establishing the initiation of the transcription in the nucleotides TG (SEQ ID NO 1). In addition, other typical sequences of promoters of Gram-positive microorganisms have been identified, such as a TG sequence in the position -15 and a zone rich in adenines in the region -45. Overlapping the region -35, a *cre* sequence (5'-ATAAAACGTTTACA) of binding to the regulator protein CcpA has been identified. Following this promoter, a sequence has been identified that would act as a *rho*-independent terminator. Preceding and overlapping this last structure, there is an RAT sequence (5' GGATTGTGACTATTTAATTAGGCGAG), similar to the binding site of anti-terminator proteins to the RNA. Both the nucleotide sequence of the lactose operon as well as that of the flanking regions 5' and 3' were deposited in the EMBL data base under number Z80834.

The regulation of the lactose operon makes possible a strict and consistent control of the expression, by using regulatory elements of the *Lactobacillus.* In the presence of the inducer of the system, the lactose, the anti-terminator protein LacT, in its active form, binds to the RAT sequence and destabilizes the terminator structure enabling the expression of the operon. The use of lactose in *L. casei* is subject to a strong repression by glucose, that is, that there is no expression of the operon when the said sugar is in the medium. In such conditions, the regulator protein CcpA binds to the *cre* sequence blocking the initiation of the transcription of the operon. Moreover, the anti-termination mechanism intervenes in this repressing effect, as the protein LacT, in the presence of glucose, is found in its inactive form and thus, the transcription comes to a halt on the level of the terminator.

The use of regulatory elements such as those described for the lactose operon of *L. casei,* and as is shown in the examples included below, allows the maximum expression of the characteristic(s) in the presence of the inducer, which in the case of the operon mentioned earlier is the lactose, the majority sugar in milk and other products derived from it, such'as whey, and forms a part of this invention. At the present time, whey is easy to obtain, and thus it would involve a minimum cost and, in addition, would provide a commercial outlet for an effluent that is often discarded.

In addition, it is possible to use any fragment whatsoever of the DNA sequence which is described in this invention or variations of them obtained by mutation that substantially maintain this regulatory capacity of the genic expression through an anti-terminator protein. These variations can be determined easily through the existing knowledge of molecular biology techniques and form a part of this invention.

These regulator sequences can be used in genic constructions in which a sequence of nucleotides encoding a gene that is wished to be expressed and then introduce it into a system of recombinant expression. The system of expression can be a plasmid, such as the one developed in this invention, or any other kind of vector from among those described in the relevant literature, such as a transposon, a bacteriophage or derivatives obtained by molecular techniques. The genes that confer the desired characteristics can be expressed in multicopy under the control of these regulatory elements and can also be integrated into the chromosome, in the *lacTEGF* operon of *L. casei* by means of an integration vector developed in this invention, remaining under the control of the aforementioned regulatory elements. The strains obtained by this procedure are safe and applicable in the food industry. The use of these genic constructions and of any of these systems of expression forms a part of this invention.

The following examples show how the use of these vectors makes it possible to regulate the expression of genes of industrial interest in transformed cells. This invention expresses the gene *gusA* of *E. coli* which codes the enzyme β-glucuronidase as well as the genes (*ilvB, ilvN*) which code the catalytic and regulator sub-units of the acetohydroxy acid synthase enzyme of *Lactococcus lactis* with the overproduction of diacetyl by *L. casei*. This system of regulation of the genic expression is applicable to *L. casei* and other bacteria with homologous anti-termination systems. The use of these cells for the expression of peptides or proteins of industrial interest forms a part of this invention.

To sum up, the system of genic expression of this invention has the following characteristics:
- Induction by sugars in *Lactobacillus casei* or other bacteria without the need to introduce any mutation in the elements of the PTS system for the transport of sugars and/or regulator proteins, such as the protein CcpA and regulators with domains regulated by elements of the PTS, such as, for example, anti-terminators or transcriptional regulators.
- Use of a regulatory element of the *Lactobacillus casei* with an anti-terminator function allowing a strict control of the expression of the desired characteristic(s) by means of induction by lactose.
- When the expression of the genes of interest is not desirable, their repression is achieved by easily assimilated sugars, such as glucose or fructose.

### DETAILED EXPLANATION OF THE DRAWINGS

**Figure 1.-** Structure and regulatory elements of the lactose operon of *L. casei.*
**Figure 2.-** Construction and physical map of the vector pIAβ5lac according to the invention. The arrows indicate the direction of the transcription.
**Figure 3.-** A) Physical map of pMJ67 according to the invention. The arrows indicate the direction of the transcription. Erm, gene of resistance to erythromycin; Ap, gene of resistance to ampicillin. *ori*, origin of replication. B) synthetic nucleotide sequence in the region of 45 pb between the genes *lacG* and *lacF*. RBS, site of binding to ribosome. * stop codon TAA.
**Figure 4.-** Physical map of the plasmid pIAβ5lacgus obtained according to the invention.
**Figure 5.-** Physical map of the plasmid pMJ70 obtained according to the invention.
**Figure 6.-** Outline corresponding to the radiographic film of the southern blot analysis of the genomic DNA of the first integrant of the gene *gusA* (course 1), CECT5290 (course 2) and *L. casei* CECT 5276 (course 3). A fragment *Bam*HI of the plasmid pRV300 that carries the gene conferring resistance to erythromycin was used as the probe.
**Figure 7.-** Physical map of the plasmid pMJ87 obtained according to the invention.
**Figure 8.-** Outline corresponding to the radiographic film of the southern blot analysis of the genomic DNA of the first integrant of the genes *ilvBN* (course 1), CECT5291 (course 2) and *L. casei* CECT 5276 (course 3). The same fragment was used as the probe for the southern blot analysis described in Figure 6.
**Figure 9.-** Determination of final metabolites.

### EXAMPLES

*Lactobacillus casei* CECT 5275, CECT5276 and *Escherichia coli* DH5α are the strains used in the various cloning and integration experiments. *Lactobacillus casei* CECT 5276 cannot use the lactose, as it presents a mutation in the gene *lacF. L. casei* has grown in an MRS medium (Oxoid) (liquid and solid) and MRS fermentation (Adsa-Micro, Scharlau S.A., Barcelona, Spain) (liquid and solid) with 0.5% sugar, using the LB medium for the growth of *E. coli.* The building of the expression vector pIAβ5lac was attained by starting from the plasmids pIL253 [Simon D. and Chopin A., 1988, Biochimie 70:559-566], pACYC184 [Chang A.C.Y. and Cohen S.N., 1978, J. Bacteriol. 134:1141-1156] and pJDC9 [Chen J.D. and Morisson D.A., 1988, Gene 64:155-164]. The plasmid pRV300 [Leloup L., Ehrlich D., Zagorec M. and Morel-Deville F., 1997, Appl. Environ. Microbiol. 63:2117-2123] was used to build the integration vector pMJ67. The concentration of the antibiotics used was: erythromycin 300 µg/ml, ampicillin 100 µg/ml and 25 µg/ml of chloramphenicol for *E. coli* and 5 µg/ml, of erythromycin for *L. casei*. The general molecular techniques were performed in accordance with the protocols described by Sambrook *et al*. [Sambrook J., Fritsch E.F. and Maniatis T., 1989, Molecular Cloning: a Laboratory Manual. 2^{nd} ed. Cold Spring Harbor Laboratory, Cold Spring Harbor, New York]. The specific techniques used for *Lactobacillus*, such as purification of genomic DNA and transformation, were those described in J. Bacteriol., 1999, 181:3928-3934. The manipulations of DNA were performed in accordance with the recommendations of the manufacturers of the restriction endonucleases and modifying enzymes. The phospho-β-galactosidase and β-glucuronidase were determined in cells made permeable of *L. casei* following the procedure described in J. Bacteriol, 1983, 154:1195-1203 and in Appl. Environ. Microbiol., 1994, 60:587-593, respectively. The PCR reactions were carried out with the commercial thermostable polymerase Dynazyme [Finnzymes Oy, Riihitontuntie, Finland] or with the Expand ™ High fidelity PCR system (Roche Molecular Biochemicals). The genomic DNA of *Lactococcus lactis* MG1363 [Gasson M.J., 1983, J. Bacteriol. 154:1-9] was purified by following the procedure described for *Lactobacillus*.

### EXAMPLE 1.- Expression of the gene gusA in multicopy.

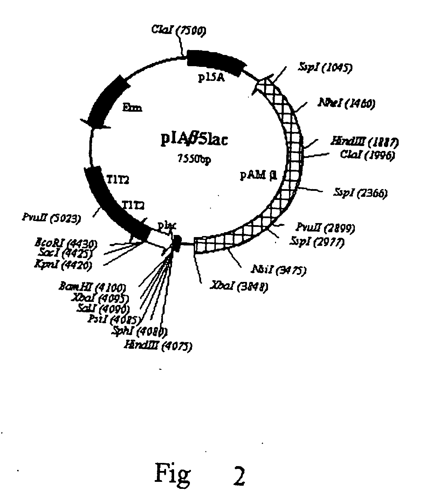

The replication vector, called pIAβ5lac, is a derivative of pIL253 and consists of a fragment of DNA with 295 pairs of bases (pb), which contains the promoter and regulator regions (*plac*) of the operon of the lactose of *L. casei,* the origin of replication for the Gram-negative bacteria (p15A) of the plasmid pACYC184, a multiple cloning site, the gene *lacZ* and two copies of the terminator *rrnB* of the ribosomal operon of *Escherichia coli* from the plasmid pJDC9 (Figure 2). This vector achieves the expression, in multicopy, of the genes cloned under *plac* in *L. casei* and bacteria with homologous anti-termination systems. These genes can code, for example, proteases, peptidases, mechanisms of resistance to bacteriophages, enzymes that intervene in the metabolism of citrate, enzymes that intervene in the production of bacteriocins or proteins that confer resistance upon bacteriocins, products of nutritional or immunological interest. The strain of *Escherichia coli* DH5β that contains pIAβ5lac has been deposited in CECT under number 5278.

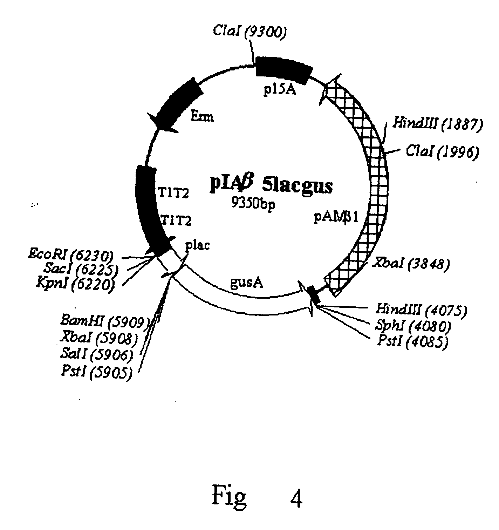

The vector of expression developed, pIAβ5lac, has been used in order to express in multicopy the gene *gusA* under the regulatory elements of the lactose operon in *L. casei.* This gene codes the enzyme β-glucuronidase of *E. coli.* For this purpose, the said gene has been enlarged by PCR, without regulator zones, taking as its mold the plasmid pNZ272 [Platteeuw C., Simons G. and de Vos W.M., 1994, Appl. Environ. Microbiol., 60:587-593]. The oligonucleotides used as primers were as follows: gus1, 5'-AAAACTGCAGTATTATTATCTTAATGAGG and gus2, 5'-CGGAATTCTCATTGTTTGCCTCCC. The sites of recognition of the restriction endonucleases (underlined) were introduced to these oligonucleotides as follows: *Pst*I to gus1 and *Eco*RI to gus 2. The enlarged DNA fragment of 1847 pb was treated with the Klenow fragment of polymerase I DNA in order to render the protuberant ends blunt and was cloned in pUC19/ *Sma*I. The construction that carried the adequate orientation was digested with the endonuclease *Pst*I and the insert was cloned in the vector pIAβ5lac previously digested with the enzyme *Pst*I. The resultant plasmid, called pIAβ5lacgus (Figure 4) was used to transform *L. casei.* One of the transformants was selected in order to perform the β-glucuronidase activity studies. In the transformant chosen it was possible to detect β-glucuronidase activity when grown on lactose, whereby the expression is being induced from the *plac* of the plasmid. The presence of glucose exercises a strong repression. Thus, the pattern of expression of the β-glucuronidase is similar to that of the lactose operon (Table 1).

**Table 1:**

| β-glucuronidase activity measured in *L. casei* CECT5275 transformed with the plasmids pIAβ5lac and pIAβ5lacgus. | |
|---|---|
| | **β-glucuronidase** **(nmol/min/mg dry wt.)** |
| *L. casei* / | **ND** |
| **pIAβ5lac R** | |
| **L** | **ND** |
| **G+L** | **ND** |
| *L. casei* / | **173.35± 29.2** |
| **pIAβ5lacgus R** | |
| **L** | **369.87± 30.6** |
| **G+L** | **ND** |

The values represent at least three independent experiments. R, ribose; L, lactose; G+L, glucose plus lactose. ND, activity not detectable.

### EXAMPLE 2.- Expression of the gene gusA integrated in the lactose operon.

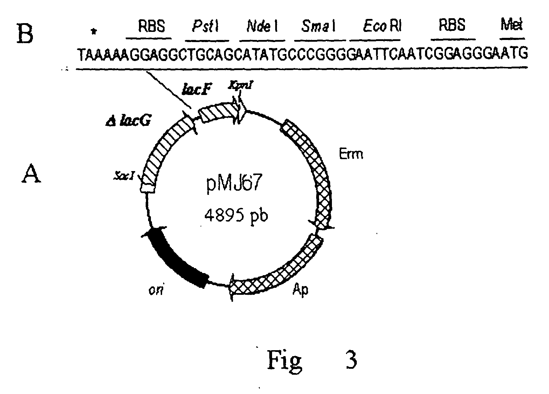

The desired products can also be obtained by inserting the respective genes in the chromosome of the bacteria under the regulatory elements selected by using chromosomal integration techniques. The simplest form of chromosomal integration is based on the use of a non-replicating vector that carries a fragment of homologous DNA to a region of the chromosome of the host bacterium, which is transformed with the said vector. The integration occurs by a Campbell type recombination mechanism or a simple crosslinking. However, this latter type is unstable, and a selective pressure must be exercised in order to keep the structure integrated. Now then, if the integrating vector carries two homologous regions, on the occurrence of two recombinations, the desired gene(s) is/are integrated in a stable manner and the vector used for the integration is eliminated. These recombinations can occur simultaneously (double crosslinking) or in a consecutive manner. In order to express the desired gene(s) starting from the regulatory elements chosen, it is advisable for integration not to take place at random, for which reason the homologous zones of the vector must be well characterized. The integration vector developed in this invention, pMJ67, enables the regulatory elements of the lactose operon of *L. casei* to be used in order to express genes of interest. This vector is based on the plasmid pRV300, to which the end 3' of the gene *lacG* and the complete gene *lacF* of the aforesaid operaton (Figure 3A) have been introduced as homologous zones, in order to direct the recombinations. The intergenic region of 45 pb that exists between the two has been replaced by a synthetic fragment of DNA that carries a site for binding to ribosome typical for *Lactobacillus* and four sites of recognition of the restriction enzymes *Pst*I, *Nde*I, *Sma*I and *Eco*RI (Figure 3B). The strain of *Escherichia coli* DH5α that contains pMJ67 has been deposited in the CECT under number 5277. The recombinations occur in *L. casei* consecutively, with the gene(s) becoming incorporated in the region of 45 pb existing between the two genes, *lacG* and *lacF*, being expressed as from the regulatory elements of the lactose operon. In order to facilitate the selection of the double recombinants, it is advisable to use a mutant copy of the gene *lacF*, which can be that carried by the integration vector or the chromosomal copy. If the gene(s) to be integrated originate from a microorganism recognized as safe ("GRAS"), the mutants of this lactic acid bacterium will be of a food grade, as, through the procedure described there will be no other DNA fragment integrated except for the gene(s) of interest, which will thus remain subject to the regulation system of the lactose operon. As the lactose is the inducer of this system of expression, the use of these regulatory elements makes possible the use of inexpensive culture mediums such as milk or its derivatives (whey) in order to produce products of technological, nutritional or pharmaceutical interest safely.

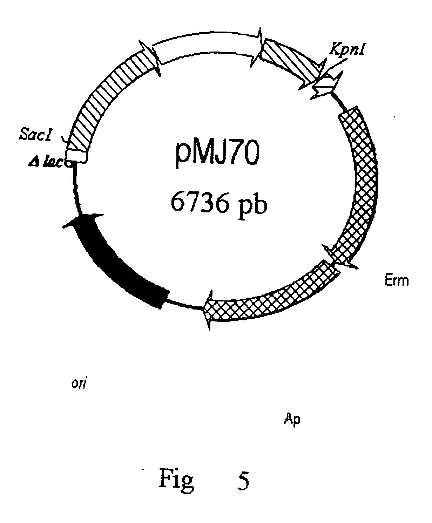

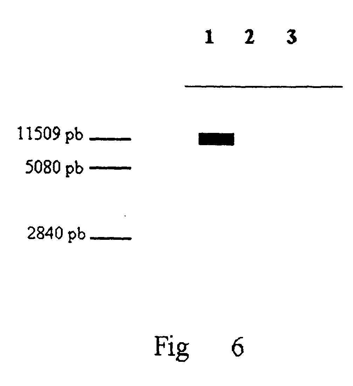

In order to demonstrate the expression in monocopy of genes, starting from the regulatory elements of the lactose operon of *L. casei,* the gene *gusA* was cloned in the integrating vector pMJ67. For this purpose, as in the preceding example, the said gene was enlarged by PCR, taking as its mold the plasmid pNZ272 and using the same oligonucleotides. In this case, the enlarged fragment was digested with the enzymes *Pst*I and *Eco*RI and was cloned in the integrating vector pMJ67 previously digested with both endonucleases. The plasmid obtained, called pMJ70 (Figure 5), was used in order to transform *L. casei* CECT5276 by electroporation. The transformants were selected on plates of the MRS medium with erythromycin. By this procedure, the colonies grown should have integrated the vector by recombination between homologous zones, as it carries the gene that codes the resistance to that antibiotic. As we mentioned earlier, pMJ67 carries two zones homologous to the bacterial genome: the end 3' of the gene *lacG* and *lacF*, whereby the simple crosslinking will occur through one of the two. An analysis was made of different transformants by PCR and an initial integrant was selected in which the recombination had occurred through *lasF*, being resistant to erythromycin and unable to ferment the lactose. In order to favor the second crosslinking, the aforementioned integrant was grown in the MRS medium without erythromycin during 200 generations. The double recombinants were selected on MRS fermentation plates with lactose and without erythromycin. Those colonies, in which the lactose operon was reconstituted, with the gene *gusA* being integrated into it, and which have lost the integrating vector, will be able to grow on lactose and will be sensitive to erythromycin. One of these mutants, deposited in the CECT under number 5290, was selected, and a southern blot analysis was performed in order to check that the resistance to erythromycin had been lost with the vector following the double recombination (Figure 6). For this purpose, the genomic DNAs of *L. casei* CECT5276, of the first integrant and of CECT5290 were digested with the endonuclease *Bgl*II, and a fragment *BAM*Hl of the plasmid pRV300 containing the gene of the erythromycin was used as the probe. A signal of hybridization was only detected in the first integrant, confirming that the mutant CECT5290 does not contain plasmidic DNA. The mutants of *L. casei* obtained only have the gene *gusA* integrated in the chromosome, which is expressed starting from the regulatory elements of the lactose operon of *L. casei.* As shown on Table 2, the β-glucuronidase activity is induced by lactose and is repressed by glucose, the same as occurs with the P-β-galactosidase activity, an enzyme coded in the lactose operon.

**Table 2:**

| P-β-galactosidase and β-glucuronidase activity measured in *L. casei* CECT5275 and CECT5290. | | |
|---|---|---|
| | **P-β-galactosidase (nmol/min/mg dry wt.)** | **β-glucuronidase (nmol/min/mg dry wt.)** |
| *L.casei* **CECT5275** **R** | **2.03 ± 0.86** | **0.98 ± 0.15** |
| **L** | **23.91 ± 2.93** | **1.89 ± 0.19** |
| **G+L** | **0.78 ± 0.24** | **1.16 ± 0.23** |
| *L.casei* **CECT5290** **R** | **10.40 ± 1.69** | **43.13 ± 11.64** |
| **L** | **57.84 ± 8.51** | **119.05 ± 8.91** |
| **G+L** | **1.26 ± 0.12** | **1.23 ± 0.20** |

The values represent at least three independent experiments. R, ribose; L, lactose; G+L, glucose plus lactose.

### EXAMPLE 3.- Expression of the genes ilvBN integrated in the lactose operon.

The procedure described enables the integration and expression of genes as from the regulatory elements of the lactose operon of *L. casei*. If the genes that are integrated originate from GRAS microorganisms, the mutants of *L. casei* obtained are safe and applicable in the food industry.

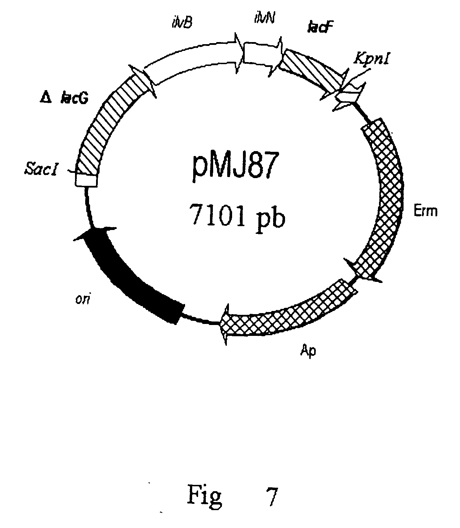

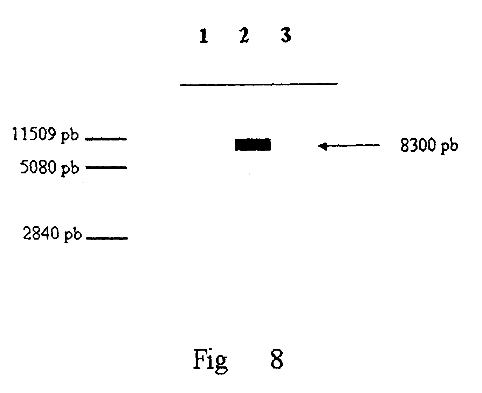

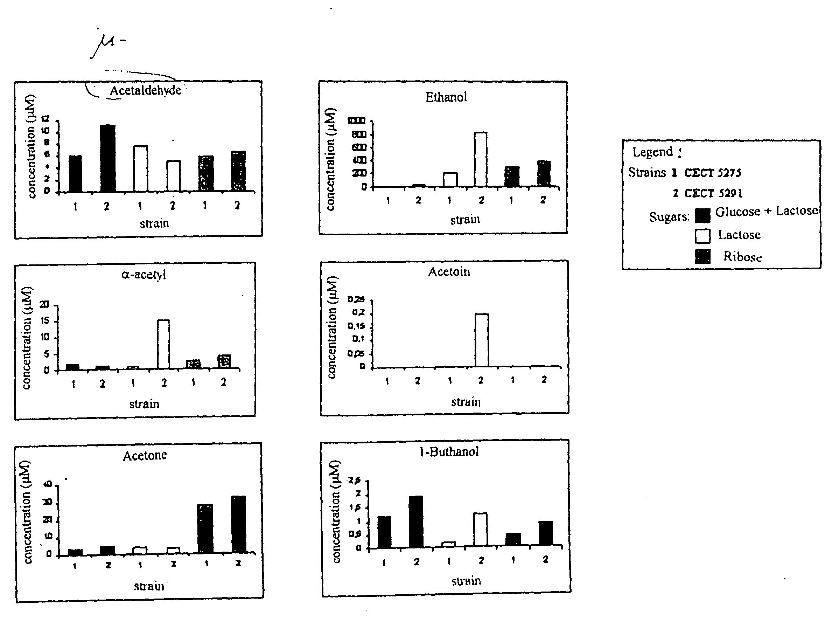

The genes *ilvBN* were enlarged by PCR taking the genomic DNA of *Lactococcus lactis* MG1363 as the mold and using the oligonucleotides ilvBN1 ( 5'-CGATCATATGAAAAAAATAAAGTTAGAAAAACCTACTTCC) and ilvBN2 (5'-CCGAATTCTTAGCCACGCTCAAAACCTGC) as primers. The same as in the preceding example, the sites of recognition of the endonucleases (underlined) have been introduced: *Ndel* at ilvBNll and *EcoRI* at ilvBN2. These genes, ilvBN, code the catalytic and regulator subunit of the acetohydroxy acid synthase enzyme involved in the biosynthesis of branched amino acids such as isoleucine and valine. This enzyme catalyzes the reaction of pyruvic acid to α-acetolactate, precursor of the diacetyl. This last product contributes considerably to the development of the aroma and flavor of fermented dairy products. The enlarged fragment of 2212 pb was cloned in the vector pMJ67, digesting both types of DNA with *Nde*l and *EcoR*I and obtaining the plasmid pMJ87 (Figure 7). The integration and selection procedure for the first integrant and of the double recombinant was the same as described earlier. In this example, a food-grade mutant of *L. casei* is obtained, which has been deposited in the CECT under number 5291, as the genes ilvBN are from *L. lactis*, a microorganism considered to be GRAS, and the vector has been lost following the second process of recombination. As shown in Figure 8, there is only hybridation between the probe described in the preceding example and the chromosomal DNA digested with the restriction enzymes *Bgl*II and *Pst*I of the first integrant. In *L. casei* CECT5291, the plasmid carrying the resistance to erythromycin has been lost following the second recombination. With the integration of these genes, it was intended to increase the production of diacetyl in *L. casei* in conditions of induction, growth in lactose. By means of gas chromatography coupled with mass spectroscopy and in accordance with the technique of suspension of cells in repose described by Danneau P. and Pérez-Martinez G. [Journal of Chromatography A, 1997, 775:225-230]. The mutant CECT5291 produces diacetyl and acetoin only under conditions of induction (Figure 9), with the production of these metabolites being at a minimum in the wild strain. Thus, with the integration of the genes *ilvBN* in the lactose operon of *L. casei*, an increase in its production has been achieved, which reaches a maximum in the presence of lactose and the absence of glucose.

LIST OF SEQUENCES

<110> CONSEJO SUPERIOR DE INVESTIGACIONES CIENTIFICAS

<120> Use of the nucleotide sequence of the promoter of the lactose operon of
lactobacillus casei in order to regulate the genic expression through an anti-
terminator protein.

<130> Anti-terminator sequence

<140>
<141>

<160> 1

<170> Patent in Ver. 2.1

<210> 1
<211> 500
<212> DNA
<213> Lactobacillus casei

<220>
<221> -10_signal
<222> (368) . . (373)

<220>
<221> -35_signal
<222> (345) . . (350)

<220>
<221> terminator
<222> (423) . . (434)

<220>
<221> terminator
<222> (439) . . (452)

<220>
<221> transcription start
<222> (379) . . (380)

<220>
<221> RAT sequence
<222> (401) . . (429)

<220>
<221> RBS
<222> (482) . . (487)

<220>
<221> cre sequence
<222> (337) . . (350)

<400> SEQ ID NO 1

ggcatcgtcg atgagccaaa gttcaaaatc gattgtttgt tgctggatac tttttaatgt 60
ctcaggcaga taatgaccta aattgtaagt tggcacaacg acggttaaat ctaagttcga 120
cgtaattggc tccttgaagc tcgttttgat tattatcgtc aaattaacgg aggcgtcaaa 180
taaagtgatt cgtttgtaga gacatgacta gcactgatca ttaaagaaca aaaggcaaca 240
cagataattg tcctaaaaaa ctgacgatgg cttgctggat ggacactgtt gaaaaagaaa 300
aaagaacagc aaactaaaga tgctacaaaa aataaaataa aacgtttaca ttctgttcag 360
attatggtac aacttgtttg ttgataggaa tattcaatgt ggattgtgac tatttaatta 420
ggcgagacca caaatcgcag tgctgatcgc agcgcgtgat ttgtggtttt ttctttgcac 480
tgggagggga tgacaacttg 500

## Claims

1. Use of the nucleotide sequence of the promoter of the lactose operon of *Lactobacillus casei* to regulate the genic expression through an anti-terminator protein.

2. Use according to claim 1 **characterized in that** the nucleotide sequence is SEQ ID NO 1.

3. Use according to claims 1 and 2 **characterized in that** the nucleotide sequence is a mutated form or a fragment that maintains substantially the same capacity.

4. Use according to claims 1 and 2 **characterized in that** the nucleotide sequence is a homologous sequence that maintains substantially the same capacity.

5. Use according to any of the claims from 1 to 4 **characterized in that** the nucleotide sequence is part of a genic construction that also contains the nucleotide sequence coding for a peptide of interest.

6. Use according to claim 5 **characterized in that** the genic construction is integrated in a system of recombinant expression with the elements necessary for the expression of a gene of interest.

7. Use according to claim 6 **characterized in that** the system of expression is, among others, a plasmid, a transposon, a bacteriophage or derivatives obtained by molecular techniques.

8. Use according to claim 7 **characterized in that** the plasmid is pIAbeta5lac.

9. Use according to claims 6 and 7 **characterized in that** the system of expression permits the integration of the nucleotide sequence in the bacterial genome.

10. Use according to claim 9 **characterized in that** the plasmid is pMJ67.

11. Use according to any of the claims from 6 to 10 **characterized in that** the system of expression is contained in a'transformed cell.

12. Use according to claim 11 **characterized in that** the cell transformed belongs, among others, to the following groups: *E. coli, Lactobacillus, Bacillus and Lactococcus.*

13. Use according to claim 12 **characterized in that** the cell transformed is CECT 5277, CECT 5278, CECT 5290 and CECT 5291.

14. Use according to any of the claims from 11 to 13 **characterized in that** the cells transformed make possible the expression regulated by lactose of a peptide of interest.

15. Use according to claim 14 **characterized in that** the peptide of interest is the enzyme acetohydroxy acid synthase.
